# FASCICULE DE BREVET EUROPEEN

(11) **EP 2 443 086 B1**
(45) Date de publication et mention de la délivrance du brevet: **22.01.2014**
(21) Numéro de dépôt: 10724794.2
(22) Date de dépôt: 07.06.2010
(51) Int. Cl.: C07C 253/10, C07C 255/01

(54) **PROCEDE DE FABRICATION DE COMPOSES NITRILES A PARTIR DE COMPOSES A INSATURATION ETHYLENIQUE**
VERFAHREN ZUR HERSTELLUNG VON NITRILVERBINDUNGEN AUS ETHYLENISCH UNGESÄTTIGTEN VERBINDUNGEN
METHOD FOR THE PRODUCTION OF NITRILE COMPOUNDS FROM ETHYLENICALLY-UNSATURATED COMPOUNDS

(30) Priorité: 16.06.2009 FR 0954015
(43) Date de publication de la demande: 25.04.2012
(73) Titulaire: Rhodia Opérations, 93306 Aubervilliers (FR)
(72) Inventeur: MASTROIANNI, Sergio, F-69006 Lyon (FR); PRINGLE, Paul, Bristol, Wiltshire BS8 3DG (GB); GARLAND, Michael, Bradford-On-Avon, Wiltshire BA15 1UT (GB); HOPEWELL, Jonathan, Wakefield, Yorkshire WF4 3JH (GB)
(74) Mandataire: Chatelan, Florence Anne
(86) Numéro de dépôt international: PCT/EP2010/057922
(87) Numéro de publication internationale: WO 2010/145960

(56) Documents cités:
- DE-A1- 19 740 180
- GB-A- 1 178 950
- US-A- 5 840 647
- US-A1- 2004 054 211
- DOWNING ET AL: "General Routes to Alkyl Phosphatrioxaadamante Ligands" ORGANOMETALLICS, vol. 27, 11 juin 2008 (2008-06-11), pages 3216-3224, XP002561941 cité dans la demande
- M. CARREIRA ET AL.: "Anatomy of Phobanes. Diastereoselective Synthesis of the Three Isomers of n-Butylphobane and a Comparison of their Donor Properties" JOURNAL OF THE AMERICAN CHEMICAL SOCIETY., vol. 131, no. 8, 30 janvier 2009 (2009-01-30), pages 3078-3092, XP002601663 ISSN: 0002-7863 cité dans la demande

## Description

La présente invention concerne un procédé d'hydrocyanation de composés organiques à insaturation éthylénique en composés comprenant au moins une fonction nitrile.

Elle se rapporte plus particulièrement à l'hydrocyanation de dioléfines telles que le butadiène ou d'oléfines substituées telles que des alcènes-nitriles comme les pentènenitriles.

Le brevet français n° 1 599 764 (ou GB 1,178,950) décrit un procédé de préparation de nitriles par addition d'acide cyanhydrique sur des composés organiques ayant au moins une double liaison éthylénique, en présence d'un catalyseur comprenant du nickel et un ligand organophosphoré, une triarylphosphite. Cette réaction peut être conduite en présence ou non d'un solvant. Des ligands de type arylphosphine ou arylarsine tertiaire étaient connus de US 2,571,099.

Quand un solvant est utilisé, il s'agit de préférence d'un hydrocarbure, tel que le benzène ou les xylènes ou d'un nitrile tel que l'acétonitrile.

Le catalyseur mis en oeuvre est un complexe organique de nickel, contenant des ligands tels que les phosphines, les arsines, les stibines, les phosphites, les arsénites ou les antimonites.

La présence d'un promoteur pour activer le catalyseur, tel qu'un composé du bore ou un sel métallique, généralement un acide de Lewis, est également préconisée dans ledit brevet.

De nombreux autres systèmes catalytiques ont été proposés comprenant généralement des composés organophosphorés appartenant à la famille des phosphites, phosphonites, phosphinites et phosphines. Ces composés organophosphorés peuvent comprendre un atome de phosphore par molécule et sont qualifiés de ligands monodentates. Ils peuvent comprendre plusieurs atomes de phosphore par molécules, ils sont alors appelés ligands pluridentates, plus particulièrement de nombreux ligands contenant deux atomes de phosphore par molécule (ligand bidentate) ont été décrits dans de nombreux brevets.

Toutefois, la recherche de nouveaux systèmes catalytiques plus performants tant en activité catalytique qu'en stabilité est toujours entreprise.

Un des buts de la présente invention est de proposer une nouvelle famille de ligands qui permet d'obtenir avec les métaux de transition des systèmes catalytiques présentant une bonne activité catalytique dans la réaction d'hydrocyanation.

A cet effet, la présente invention propose un procédé d'hydrocyanation d'un composé hydrocarboné comprenant au moins une insaturation éthylénique par réaction en milieu liquide avec le cyanure d'hydrogène en présence d'un catalyseur comprenant un élément métallique choisi parmi les métaux de transition et un ou des ligands organophosphorés caractérisé en ce que le ligand organophosphoré comprend au moins un composé répondant à la formule générale (II) : 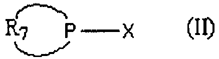 dans lesquelles:
- R₇ représente un radical divalent de formule générale III suivante : 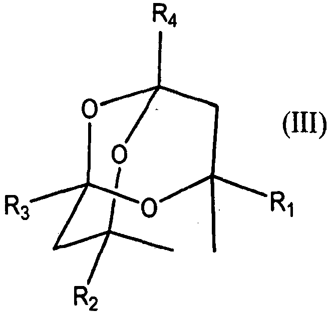 ou un radical divalent de formule générale (IV) suivante : 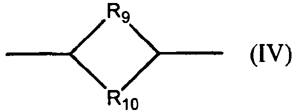 dans laquelle R₉ et R₁₀ identiques ou différents représentent un radical divalent aliphatique linéaire ou ramifié ayant de 1 à 6 atomes de carbone
   R₁, R₂, R₃, R₄ identiques ou différents représentent un atome d'hydrogène, un radical alkyl linéaire ou ramifié ayant de 1 à douze atomes de carbone pouvant contenir des hétéroatomes, un radical comprenant un radical aromatique ou cycloaliphatique substitué ou non pouvant comprendre des hétéroatomes, un radical carbonyle, alcoxycarbonyle ou alcoxy, un atome d'halogène, un groupement nitrile ou un groupement halogénoalkyle ayant 1 à 12 atomes de carbone
- X représente un atome d'halogène choisi dans le groupe comprenant le fluor et le brome,

Avantageusement, R₁, R₂, R₃, R₄ identiques ou différents représentent un atome d'hydrogène, un radical alkyl linéaire ou ramifié ayant de 1 à 12 atomes de carbone pouvant contenir des hétéroatomes.

Avantageusement, le ligand organophosphoré est un composé correspondant à la formule générale (II) avec le radical X représentant le fluor et le radical R₇ correspondant à la formule (III) ou (IV).

Les ligands préférés de l'invention répondent aux formules chimiques suivantes :

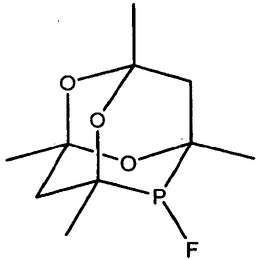

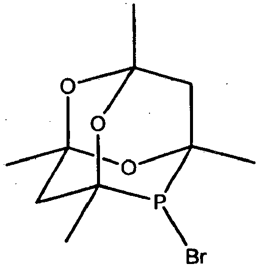

Ces composés et leur méthode de fabrication ont été décrits dans plusieurs communications ou publications scientifiques. On peut citer, à titre d'exemple, la publication de Downing et al. Organometallics, 2008, vol.27 n°13, pages 3216-3224.

D'autres ligands préférés de l'invention répondent aux formules chimiques suivantes :

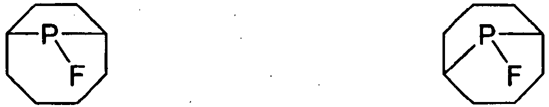

Les ligands organophosphorés (fluorophosphites) répondant à la formule (II) convenables pour l'invention sont notamment décrits dans la demande de brevet US20080081759.

Selon l'invention, la composition du système catalytique peut être représentée par la formule générale (V) (cette formule ne correspond pas à la structure des composés et complexes présents dans le système catalytique):

M[L_{f}]ₜ (V)

Dans laquelle:
M est un métal de transition
L_{f} représente au moins un ligand organophosphoré de formule (II)
t représente un nombre compris entre 1 et 10 (bornes incluses)

Dans un mode de réalisation de l'invention, le ligand L_{f} est un mélange de composés organophosphorés dont au moins un est un composé correspondant à la formule générale (II). Le mélange peut comprendre par exemple un composé organophosphite monodentate tel que le tritolylphosphite (TTP) ou le triphénylphosphite (TPP).

Dans la suite de la description, le terme composé organophosphoré désigne aussi bien les composés de formule (II) qu'un mélange de composés organophosphorés comprenant par exemple un composé monodentate organophosphite et au moins un composé de formule (II).

Les métaux M qui peuvent être complexés sont de manière générale tous les métaux de transition des groupes 1b, 2b, 3b, 4b, 5b, 6b, 7b et 8 de la Classification périodique des éléments, telle que publiée dans "Handbook of Chemistry and Physics, 51 st Edition (1970-1971)" de The Chemical Rubber Company.

Parmi ces métaux, on peut citer plus particulièrement les métaux pouvant être utilisés comme catalyseurs dans les réactions d'hydrocyanation. Ainsi on peut mentionner à titre d'exemples non limitatifs, le nickel, le cobalt, le fer, le ruthénium, le rhodium, le palladium, l'osmium, l'iridium, le platine, le cuivre, l'argent, l'or, le zinc, le cadmium, le mercure. Le nickel est l'élément préféré pour l'hydrocyanation des oléfines et des nitriles insaturés.

La préparation des systèmes catalytiques comprenant des composés organophosphorés selon l'invention peut être effectuée en mettant en contact une solution d'un composé du métal choisi, par exemple le nickel, avec une solution du composé organophosphoré de l'invention.

Le composé du métal peut être dissous dans un solvant. Le métal peut se trouver dans le composé mis en oeuvre, soit au degré d'oxydation qu'il aura dans le complexe organométallique, soit à un degré d'oxydation supérieur.

A titre d'exemple, on peut indiquer que dans les complexes organométalliques de l'invention, le rhodium est au degré d'oxydation (I), le ruthénium au degré d'oxydation (II), le platine au degré d'oxydation (0), le palladium au degré d'oxydation (0), l'osmium au degré d'oxydation (II), l'iridium au degré d'oxydation (I), le nickel au degré d'oxydation (0).

Si lors de la préparation du complexe organométallique, le métal est mis en oeuvre à un degré d'oxydation plus élevé, il pourra être réduit in situ.

Parmi les composés de métaux M utilisables pour la préparation des complexes organométalliques on peut citer, à titre d'exemples non limitatifs, les composés suivants du nickel :
- les composés dans lesquels le nickel est au degré d'oxydation zéro comme le tétracyanonickelate de potassium K₄ [Ni(CN)₄], le bis (acrylonitrile) nickel zéro, le bis (cyclooctadiène-1,5) nickel (appelé également Ni(cod)₂) et les dérivés contenant des ligands comme le tétrakis (triphényl phosphine) nickel zéro.
- les composés du nickel comme les carboxylates (notamment l'acétate), carbonate, bicarbonate, borate, bromure, chlorure, citrate, thiocyanate, cyanure, formiate, hydroxyde, hydrophosphite, phosphite, phosphate et dérivés, iodure, nitrate, sulfate, sulfite, aryl- et alkyl-sulfonates.

Quand le composé du nickel utilisé correspond à un état d'oxydation du nickel supérieur à 0, on ajoute au milieu réactionnel un réducteur du nickel réagissant préférentiellement avec celui-ci dans les conditions de la réaction. Ce réducteur peut être organique ou minéral. On peut citer comme exemples non limitatifs les borohydrures comme le Na BH₄, le K BH₄, la poudre de Zn, le magnésium ou l'hydrogène

Quand le composé du nickel utilisé correspond à l'état d'oxydation 0 du nickel, on peut également ajouter un réducteur du type de ceux précités, mais cet ajout n'est pas impératif

Quand on utilise un composé du fer, les mêmes réducteurs conviennent. Dans le cas du palladium, les réducteurs peuvent être, en outre, des éléments du milieu réactionnel (phosphine, solvant, oléfine).

Les composés organiques comportant au moins une double liaison éthylénique plus particulièrement mis en oeuvre dans le présent procédé sont des dioléfines comme le butadiène, l'isoprène, l'hexadiène-1,5, le cyclooctadiène-1,5, les nitriles aliphatiques à insaturation éthylénique, particulièrement les pentène-nitriles linéaires comme le pentène-3-nitrile, le pentène-4-nitrile, les monooléfines comme le styrène, le méthyl-styrène, le vinyl-naphtalène, le cyclohexène, le méthyl-cyclohexène ainsi que les mélanges de plusieurs de ces composés.

Les pentène-nitriles peuvent contenir, en plus du pentène-3-nitrile et du pentène-4-nitrile, des quantités, généralement minoritaires, d'autres composés, comme le méthyl-2-butène-3-nitrile, le méthyl-2-butène-2-nitrile, le pentène-2-nitrile, le valéronitrile, l'adiponitrile, le méthyl-2-glutaronitrile, l'éthyl-2-succinonitrile ou le butadiène, provenant par exemple de la réaction antérieure d'hydrocyanation du butadiène en nitriles insaturés.

En effet, lors de l'hydrocyanation du butadiène, il se forme avec les pentène-nitriles linéaires des quantités non négligeables de méthyl-2-butène-3-nitrile et de méthyl-2-butène-2-nitrile.

Le système catalytique utilisé pour l'hydrocyanation selon le procédé de l'invention peut être préparé avant son introduction dans la zone de réaction, par exemple par addition au(x) composé(s) organophosphoré(s) seul(s) ou dissous dans un solvant, la quantité appropriée de composé du métal de transition choisi et éventuellement du réducteur. Il est également possible de préparer le système catalytique "in situ" par simple addition du ou des composés organophosphorés et du composé du métal de transition dans le milieu réactionnel d'hydrocyanation avant ou après l'addition du composé à hydrocyaner.

La quantité de composé du nickel ou d'un autre métal de transition utilisée est choisie pour obtenir une concentration en mole de métal de transition par mole de composés organiques à hydrocyaner comprise entre 10⁻⁴ et 1, et de préférence entre 0,005 et 0,5 mole de nickel ou de l'autre métal de transition mis en oeuvre.

La quantité de composés organophosphorés utilisée pour former le catalyseur est choisie de telle sorte que le nombre de moles de ce composé rapporté à 1 mole de métal de transition soit de 0,5 à 100 et de préférence de 2 à 50.

Bien que la réaction soit conduite généralement sans solvant, il peut être avantageux de rajouter un solvant organique inerte. Le solvant peut être un solvant du catalyseur qui est miscible à la phase comprenant le composé à hydrocyaner à la température d'hydrocyanation. A titre d'exemples de tels solvants, on peut citer les hydrocarbures aromatiques, aliphatiques ou cycloaliphatiques.

La réaction d'hydrocyanation est généralement réalisée à une température de 10°C à 200°C et de préférence de 30°C à 120°C. Elle peut être réalisée en milieu monophasique.

Le procédé de l'invention peut être mis en oeuvre de manière continue ou discontinue.

Le cyanure d'hydrogène mis en oeuvre peut être préparé à partir des cyanures métalliques, notamment le cyanure de sodium, ou des cyanhydrines, comme la cyanhydrine de l'acétone ou par tout autre procédé de synthèse connu tel que le procédé Andrussov consistant à faire réagir le méthane avec l'ammoniac et de l'air.

Le cyanure d'hydrogène exempt d'eau est introduit dans le réacteur sous forme gazeuse ou sous forme liquide. Il peut également être préalablement dissous dans un solvant organique.

Dans le cadre d'une mise en oeuvre discontinue, on peut en pratique charger dans un réacteur, préalablement purgé à l'aide d'un gaz inerte (tel qu'azote, argon), soit une solution contenant la totalité ou une partie des divers constituants tels que les composés organophosphorés conformes à l'invention, le composé de métal de transition (le nickel), les éventuels réducteur et solvant, soit séparément lesdits constituants. Généralement le réacteur est alors porté à la température choisie, puis le composé à hydrocyaner est introduit. Le cyanure d'hydrogène est alors lui-même introduit, de préférence de manière continue et régulière.

Quand la réaction (dont on peut suivre l'évolution par dosage de prélèvements) est terminée, le mélange réactionnel est soutiré après refroidissement et les produits de la réaction sont isolés et séparés, par exemple, par distillation.

Avantageusement, la synthèse de dinitriles tels que l'adiponitrile à partir de dioléfines (butadiène) est obtenue en deux étapes successives. La première étape consiste à hydrocyaner une double liaison de la dioléfine pour obtenir un mononitrile insaturé. La seconde étape consiste à hydrocyaner l'insaturation du mononitrile pour obtenir le ou les dinitriles correspondants. Ces deux étapes sont généralement mises en oeuvre avec un système catalytique comprenant un complexe organométallique de même nature. Toutefois, les rapports composé organophosphoré/ élément métallique et concentration du catalyseur peuvent être différents. En outre, il est préférable d'associer au système catalytique un cocatalyseur ou promoteur dans la seconde étape. Ce cocatalyseur ou promoteur est généralement un acide de Lewis.

L'acide de Lewis utilisé comme cocatalyseur permet notamment, dans le cas de l'hydrocyanation des nitriles aliphatiques à insaturation éthylénique, d'améliorer la linéarité des dinitriles obtenus, c'est-à-dire le pourcentage de dinitrile linéaire par rapport à la totalité des dinitriles formés, et/ou d'augmenter l'activité et la durée de vie du catalyseur.

Par acide de Lewis, on entend dans le présent texte, selon la définition usuelle, des composés accepteurs de doublets électroniques.

On peut mettre en oeuvre notamment les acides de Lewis cités dans l'ouvrage édité par G.A. OLAH "Friedel-Crafts and related Reactions", tome I, pages 191 à 197 (1963).

Les acides de Lewis qui peuvent être mis en oeuvre comme cocatalyseurs dans le présent procédé sont choisis parmi les composés des éléments des groupes Ib, IIb, IIIa, IIIb, IVa, IVb, Va, Vb, Vlb, VIIb et VIII de la Classification périodique des éléments. Ces composés sont le plus souvent des sels, notamment des halogénures, comme chlorures ou bromures, sulfates, sulfonates, halogenosulfonates, perhalogénoalkyl sulfonates, notamment fluoroalkylsulfonates ou perfluoroalkylsulfonates, carboxylates et phosphates.

A titre d'exemples non limitatifs de tels acides de Lewis, on peut citer le chlorure de zinc, le bromure de zinc, l'iodure de zinc, le chlorure de manganèse, le bromure de manganèse, le chlorure de cadmium, le bromure de cadmium, le chlorure stanneux, le bromure stanneux, le sulfate stanneux, le tartrate stanneux, le trifluorométhylsulfonate d'indium, les chlorures ou bromures des éléments des terres rares comme le lanthane, le cérium, le praséodyme le néodyme, le samarium, l'europium, le gadolinium, le terbium, le dysprosium, l'hafnium, l'erbium, le thallium, l'ytterbium et le lutétium, le chlorure de cobalt, le chlorure ferreux, le chlorure d'yttrium.

On peut également utiliser comme acide de Lewis des composés organométalliques comme le triphénylborane, l'isopropylate de titane ou les composés décrits dans les demandes de brevet français FR 2.926.816 déposée le 25 janvier 2008 et FR 2.937.321 déposée le 21 octobre 2008.

On peut bien entendu mettre en oeuvre des mélanges de plusieurs acides de Lewis comme cela est décrit dans la demande de brevet français FR 2.941.455 déposée le 29 janvier 2009.

Parmi les acides de Lewis, on préfère tout particulièrement le chlorure de zinc, le bromure de zinc, le chlorure stanneux, le bromure stanneux le triphénylborane et les mélanges chlorure de zinc/chlorure stanneux, l'anhydride diphénylborinique et le tétraisobutyl dialuminoxane.

Le cocatalyseur acide de Lewis mis en oeuvre représente généralement de 0,01 à 50 moles par mole de composé de métal de transition, plus particulièrement de composé du nickel, et de préférence de 1 à 10 mole par mole.

Les mononitriles insaturés mis en oeuvre dans cette deuxième étape sont avantageusement des pentène-nitriles linéaires comme le pentène-3-nitrile, le pentène-4-nitrile et leurs mélanges.

Ces pentène-nitriles peuvent contenir des quantités, généralement minoritaires, d'autres composés, comme le méthyl-2-butène-3-nitrile, le méthyl-2-butène-2-nitrile, le pentène-2-nitrile.

La solution catalytique utilisée pour l'hydrocyanation en présence d'acide de Lewis peut être préparée avant son introduction dans la zone de réaction, par exemple par addition aux composés organophosphorés, de la quantité appropriée de composé du métal de transition choisi, de l'acide de Lewis et éventuellement du réducteur. Il est également possible de préparer la solution catalytique "in situ" par simple addition de ces divers constituants dans le milieu réactionnel.

Il est également possible dans les conditions du procédé d'hydrocyanation de la présente invention, et notamment en opérant en présence du catalyseur décrit précédemment comportant au moins un composé organophosphoré conforme à l'invention et au moins un composé d'un métal de transition, de réaliser, en absence de cyanure d'hydrogène, l'isomérisation du méthyl-2-butène-3-nitrile en pentènenitriles, et plus généralement des nitriles insaturés ramifiés en nitriles insaturés linéaires.

Le méthyl-2-butène-3-nitrile soumis à l'isomérisation peut être mis en oeuvre seul ou en mélange avec d'autres composés. Ainsi on peut engager du méthyl-2-butène-3-nitrile en mélange avec du méthyl-2-butène-2 nitrile, du pentène-4-nitrile, du pentène-3-nitrile, du pentène-2-nitrile, du butadiène.

Il est particulièrement intéressant de traiter le mélange réactionnel provenant de l'hydrocyanation du butadiène par l'acide cyanhydrique en présence d'au moins un composé organophosphoré conforme à l'invention et d'au moins un composé d'un métal de transition, plus préférentiellement d'un composé du nickel au degré d'oxydation 0, tel que défini précédemment.

Dans le cadre de cette variante préférée, le système catalytique étant déjà présent pour la réaction d'hydrocyanation du butadiène, il suffit d'arrêter toute introduction de cyanure d'hydrogène, pour laisser se produire la réaction d'isomérisation.

On peut, le cas échéant, dans cette variante faire un léger balayage du réacteur à l'aide d'un gaz inerte comme l'azote ou l'argon par exemple, afin de chasser l'acide cyanhydrique qui pourrait être encore présent.

La réaction d'isomérisation est généralement réalisée à une température comprise entre 10°C et 200°C et de préférence entre 60°C et 140°C.

Dans le cas préféré d'une isomérisation suivant immédiatement la réaction d'hydrocyanation du butadiène, il sera avantageux d'opérer à la température à laquelle l'hydrocyanation a été conduite ou légèrement supérieure.

Comme pour le procédé d'hydrocyanation de composés à insaturation éthylénique, le système catalytique utilisé pour l'isomérisation peut être préparé avant son introduction dans la zone de réaction, par exemple par mélange du ou des composés organophosphorés, de la quantité appropriée de composé du métal de transition choisi et éventuellement du réducteur. Il est également possible de préparer le système catalytique "in situ" par simple addition de ces divers constituants dans le milieu réactionnel. La quantité de composé du métal de transition et plus particulièrement du nickel utilisée, ainsi que la quantité de composé organophosphorés sont les mêmes que pour la réaction d'hydrocyanation.

Bien que la réaction d'isomérisation soit conduite généralement sans solvant, il peut être avantageux de rajouter un solvant organique inerte qui pourra être utilisé comme solvant d'extraction, ultérieurement. C'est notamment le cas lorsqu'un tel solvant a été mis en oeuvre dans la réaction d'hydrocyanation du butadiène ayant servi à préparer le milieu soumis à la réaction d'isomérisation. De tels solvants peuvent être choisis parmi ceux qui ont été cités précédemment pour l'hydrocyanation.

Toutefois, la préparation de composés dinitriles par hydrocyanation d'une oléfine comme le butadiène peut être réalisée en utilisant un système catalytique conforme à l'invention pour les étapes de formation des nitriles insaturés et l'étape d'isomérisation ci-dessus, la réaction d'hydrocyanation des nitriles insaturés en dinitriles pouvant être mis en oeuvre avec un système catalytique conforme à l'invention ou tout autre système catalytique déjà connu pour cette réaction.

De même, la réaction d'hydrocyanation de l'oléfine en nitriles insaturés et l'isomérisation de ceux-ci peuvent être réalisées avec un système catalytique différent de celui de l'invention, l'étape d'hydrocyanation des nitriles insaturés en dinitriles étant mise en oeuvre avec un système catalytique conforme à l'invention.

D'autres détails, avantages de l'invention seront illustrés par les exemples donnés ci-dessous uniquement à titre indicatif, sans caractère limitatif.

### EXEMPLES

### Abréviations utilisées

- Cod: cyclooctadiène
- Ni(Cod)₂ : bis(1,5-cyclooctadiène)nickel
- 3PN: 3-pentènenitrile
- AdN: Adiponitrile
- ESN: éthylsuccinonitrile
- MGN: méthylglutaronitrile
- DN : composés dinitriles (AdN, MGN ou ESN)
- TTP : tritolylphosphite
- TIBAO: tetraisobutyldialuminoxane
- RR(DN): rendement réel de dinitriles correspondant au rapport du nombre de moles formées de dinitriles sur le nombre de moles de 3PN engagé
- Linéarité (L): rapport du nombre de moles d'AdN formées sur le nombre de moles de dinitriles formées (somme des moles d'AdN, ESN et MGN)

Les composés suivants: 3PN, Ni(Cod)₂, ZnCl₂, TiBAO, TTP, anhydride diphénylborinique (Ph₂BOPh₂), sont des produits connus et disponibles commercialement.

### Synthèse des composés de formule générale (II) :

Un composé nommé CgPH de formule suivante : 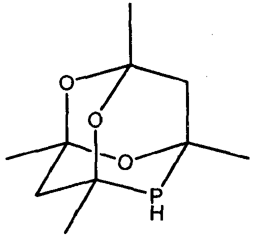 est synthétisé selon le procédé décrit dans la publication de Downing et al. Organometallics, 2008, vol.27 n°13, pages 3216-3224 ce composé est utilisé comme matière première pour la synthèse des ligands A et B de formule suivante :

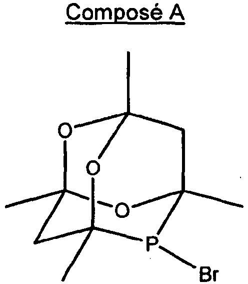

Une solution de Br₂ (3,5158g, 0,022 mol) dans CH₂Cl₂ (30 ml) est ajoutée pendant 30 minutes à une solution de composé CgPH (4,3243 g, 0,02 mol) dans CH₂Cl₂ (60 ml) à 0°C et agité à cette température pendant 30 minutes, puis pendant une heure à température ambiante. Le solvant est évaporé et un solide légèrement jaune est obtenu (Composé A). RMN ³¹P δ 53,5 (dans CH₂Cl₂)

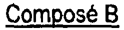

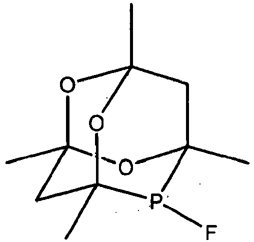

0,92 g de composé A (3,1 mmol) est ajouté à une suspension de CsF sec (2,45 g ; 16,12 mmol) dans le THF (50 ml) et le mélange est chauffé à reflux pendant 72 heures. Le mélange est ensuite filtré lors du refroidissement à température ambiante, le solvant du filtrat est évaporé sous vide et un solide blanc est ainsi obtenu. 20 ml d'hexane sont ensuite ajoutés, la suspension correspondante est filtrée, l'hexane de la solution organique est évaporé sous vide et un solide blanc est finalement obtenu (0,532 g, 73%)(Composé B).
Analyse élémentaire, trouvé (calcul) : C, 51,10 (51,28); H, 6,88 (6,89).
31 P RMN (121 MHz; C₆D₆): δP 125,4 (d, 1J(PF) 896,9 Hz).
19F RMN (282 MHz; C₆D₆): δF 209,84 (d, 1J(PF) 897,1 Hz).

Deux composés nommés Sym-PhobPCl et Asym-PhobPCl de formules suivantes : 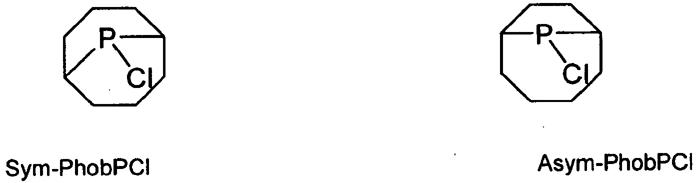 sont synthétisés selon le procédé décrit dans la publication M. Carreira, M. Charernsuk, M. Eberhard, N. Fey, R. van Ginkel, A. Hamilton, W. P. Mul, A. G. Orpen, H. Phetmung, P. G. Pringle, J. Am. Chem. Soc , 2009, 131, 3078-3092. Ces composés sont utilisés comme matière première pour la synthèse respectivement des ligands C et D de formule suivante :

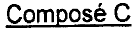

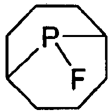

Un mélange de Sym-PhobPCl (0,500 g, 2,83 mmol) et CsF (4,31 g, 28,4 mmol) dans de l'acéfonitrile (8 ml) est chauffé à reflux pendant 1 heure. Le solvant est ensuite évaporé puis du dicholorométhane (6 ml) est ajouté. La suspension obtenue est filtrée et le solvant est évaporé sous vide.
Quantité obtenue : 0,341 g, 75%
Analyse élémentaire, trouvé (calcul): C, 59,87 (59,99); H, 8,59 (8,81)
³¹P{¹H} RMN (CDCl₃): 159,45 (d, J_{PF} = 865 Hz)

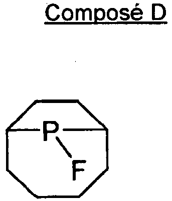

Un mélange de Asym-PhobPCl (0,500 g, 2,83 mmol) et CsF (4,31 g, 28,4 mmol) dans de l'acétonitrile (8 ml) est chauffé à reflux pendant 1 heure. Le solvant est ensuite évaporé puis du dicholorométhane (6 ml) est ajouté. La suspension obtenue est filtrée et le solvant est évaporé sous vide.
Quantité obtenue : 0,193 g, 43%
³¹P{¹H) RMN (CDCl₃): 217,33 (d, J_{PF} = 808 Hz)

### Exemples 1 à 11 : Hydrocyanation du 3-PN en AdN

Le mode opératoire général utilisé est le suivant :

Sous atmosphère d'argon, dans un tube de verre type Shott de 60 ml équipé d'un bouchon-septum, sont chargés successivement:
- le ligand (ligand A, ligand B, ligand C ou ligand D) (1 mmol, 2 équivalents en P)
- 1,21 g (15 mmol, 30 équivalents) de 3PN anhydre
- 138 mg (0,5 mmol, 1 équivalent) de Ni(cod)₂
- acide de Lewis (voir tableau 1 pour la quantité et la nature)

Le mélange est porté sous agitation à 70°C. La cyanhydrine de l'acétone est injectée dans le milieu réactionnel par un pousse-seringue à un débit de 0,45 ml par heure. Après 3 heures d'injection le pousse-seringue est arrêté. Le mélange est refroidi à température ambiante, dilué à l'acétone et analysé par chromatographie en phase gazeuse.

Les résultats sont regroupés dans le tableau 1 suivant :

**Tableau 1:**

| exemple | ligand | Acide de Lewis | Acide de Lewis/Ni (molaire) | Linéarité | RR (DN) |
|---|---|---|---|---|---|
| 1 | A | ZnCl₂ | 1 | 100 | 1,9 |
| 2 | A | TIBAO | 0,5 | 94,9 | 2,8 |
| 3 | A | Ph₂BOBPh₂ | 0,5 | 100 | 2,4 |
| 4 | B | ZnCl₂ | 1 | 65,6 | 82,6 |
| 5 | B | TIBAO | 0,5 | 69,3 | 31,5 |
| 6 | B | Ph₂BOBPh₂ | 0,5 | 84,9 | 20,6 |
| 10 | C | Ph₂BOBPh₂ | 0,5 | 81,8 | 19,2 |
| 11 | D | ZnCl₂ | 1 | 100 | 1 |

### Exemple 12 : Hydrocyanation du 3-PN en AdN

Le mode opératoire général utilisé est le suivant

Sous atmosphère d'argon, dans un tube de verre type Shott de 60 ml équipé d'un bouchon-septum, sont chargés successivement:
- 0,32 mmol de ligand
- 5 mmol de 3PN anhydre
- 0,17 mmol de Ni(cod)₂
- 0,15 mmol de ZnCl₂

Le mélange est porté sous agitation à 70°C. La cyanhydrine de l'acétone est injectée dans le milieu réactionnel par un pousse-seringue à un débit de 0,45 ml par heure. Après 3 heures d'injection le pousse-seringue est arrêté. Le mélange est refroidi à température ambiante, dilué à l'acétone et analysé par chromatographie en phase gazeuse.

Les résultats sont regroupés dans le tableau 2 suivant :

**Tableau 2:**

| exemple | ligand | Acide de Lewis | Acide de Lewis/Ni (molaire) | Linéarité | RR (DN) |
|---|---|---|---|---|---|
| 12 | C | ZnCl₂ | 0,9 | 76,4 | 12,8 |

### Exemples 13 et 14 : Hydrocyanation du 3-PN en AdN

Sous atmosphère d'argon, dans un tube de verre type Shott de 60 ml équipé d'un bouchon-septum, sont chargés successivement:
- ligand 1 (voir tableau 3 pour nature et quantité)
- ligand 2 (voir tableau 3 pour nature et quantité)
- 1,21 g (15 mmol, 30 équivalents) de 3PN
- 138 mg (0,5 mmol, 1 équivalent) de Ni(cod)₂
- acide de Lewis (voir tableau 3 pour nature et quantité)

Le mélange est porté sous agitation à 70°C. La cyanhydrine de l'acétone est injectée dans le milieu réactionnel par un pousse-seringue à un débit de 0,45 ml par heure. Après 3 heures d'injection le pousse-seringue est arrêté. Le mélange est refroidi à température ambiante, dilué à l'acétone et analyse par chromatographie en phase gazeuse.

Les résultats sont regroupés dans le tableau 3 suivant :

**Tableau 3**

| Exemple | Ligand 1 | Ligand 2 | Ligand1/Ligand2/Ni (équivalents molaire) | Acide de Lewis | Acide de Lewis/Ni (molaire) | Linéarité | RR (DN) |
|---|---|---|---|---|---|---|---|
| 13 | TTP | B | 4,5/0,5/1 | Ph₂BOBPh₂ | 0,5 | 90 | 5,2 |
| 14 comparatif | TTP | - | 5/0/1 | Ph₂BOBPh₂ | 0,5 | 73,8 | 1,2 |

## Revendications

1. Procédé d'hydrocyanation d'un composé hydrocarboné comprenant au moins une insaturation éthylénique par réaction en milieu liquide avec le cyanure d'hydrogène en présence d'un catalyseur comprenant un élément métallique choisi parmi les métaux de transition et un ligand organophosphoré **caractérisé en ce que** le ligand organophosphoré comprend au moins un composé répondant à la formule générale (II) : 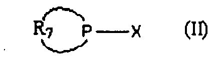 dans laquelle :
R₇ représente un radical divalent de formule générale (III) suivante : 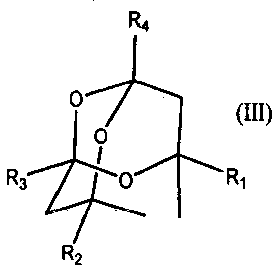 ou un radical divalent de formule générale (IV) suivante : 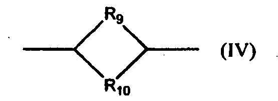 dans laquelle R₉ et R₁₀ identiques ou différents représentent un radical divalent aliphatique linéaire ou ramifié ayant de 1 à 6 atomes de carbone
R₁, R₂, R₃, R₄ identiques ou différents représentent un atome d'hydrogène, un radical alkyl linéaire ou ramifié ayant de 1 à 12 atomes de carbone pouvant contenir des hétéroatomes, un radical comprenant un radical aromatique ou cycloaliphatique substitué ou non pouvant comprendre des hétéroatomes, un radical carbonyle, alcoxycarbonyle ou alcoxy, un atome
d'halogène, un groupement nitrile ou un groupement halogénoalkyle ayant 1 à 12 atomes de carbone
X représente un atome d'halogène choisi dans le groupe comprenant le fluor et le brome

2. Procédé selon la revendication 1, **caractérisé en ce que** R₁, R₂, R₃, R₄ identiques ou différents représentent un atome d'hydrogène, un radical alkyl linéaire ou ramifié ayant de 1 à 12 atomes de carbone pouvant contenir des hétéroatomes.

3. Procédé selon l'une des revendications précédentes, **caractérisé en ce que** le composé de formule générale (II) répond à une des formules suivantes :

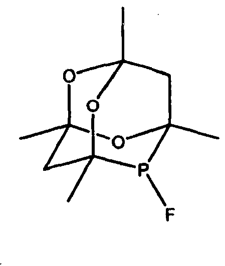

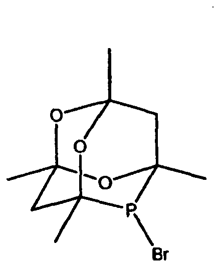

4. Procédé selon l'une des revendications 1 ou 2, **caractérisé en ce que** le composé de formule générale (II) répond à une des formules suivantes :

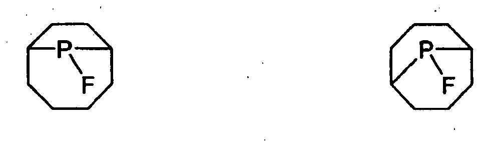

5. Procédé selon l'une des revendications précédentes, **caractérisé en ce que** l'élément métallique est choisi dans le groupe comprenant le nickel, le cobalt, le fer, le ruthénium, le rhodium, le palladium, l'osmium, l'iridium, le platine, le cuivre, l'argent, l'or, le zinc, le cadmium, le mercure.

6. Procédé selon l'une des revendications précédentes **caractérisé en ce que** la composition du système catalytique est exprimée par la formule générale (V):
M [L_{f}]ₜ (V)
Dans laquelle:
M est un métal de transition.
L_{f} représente le ou les ligands organophosphorés dont au moins un correspond à un composé de formule (II)
t représente un nombre compris entre 1 et 10 (bornes incluses)

7. Procédé selon la revendication 6 **caractérisé en ce que** L_{f} représente un mélange de ligands organophosphorés comprenant au moins un ligand correspondant à un composé de formule (II) et au moins un ligand organophosphite monodentate.

8. Procédé selon la revendication 7, **caractérisé en ce que** le ligand organophosphite monodentate est choisi dans le groupe comprenant le tritolylphosphite et le triphénylphosphite.

9. Procédé selon l'une des revendications précédentes, **caractérisé en ce que** les composés organiques comportant au moins une double liaison éthylénique sont choisis parmi les dioléfines comme le butadiène, l'isoprène, l'hexadiène-1,5, le cyclooctadiène-1,5, les nitriles aliphatiques à insaturation éthylénique, particulièrement les pentènenitriles linéaires comme le pentène-3-nitrite, le pentène-4-nitrile, les monooléfines comme le styrène, le méthyl-styrène, le vinyl-naphtalène, le cyclohexène, le méthyl-cyclohexène ainsi que les mélanges de plusieurs de ces composés.

10. Procédé selon l'une des revendications précédentes, **caractérisé en ce que** la quantité de composé du nickel ou d'un autre métal de transition utilisée est choisie de telle sorte qu'il y ait par mole de composé organique à hydrocyaner entre 10⁻⁴ et 1 mole de nickel ou de l'autre métal de transition mis en oeuvre et **en ce que** la quantité de composés organosphosphorés utilisée est choisie de telle sorte que le nombre de moles de ces composés rapporté à 1 mole de métal de transition soit de 0,5 à 100.

11. Procédé selon l'une des revendications précédentes d'hydrocyanation en dinitriles de composés nitriles à insaturation éthylénique, par réaction avec le cyanure d'hydrogène, **caractérisé en ce que** l'on opère en présence d'un système catalytique comprenant au moins un composé d'un métal de transition, au moins un composé de formule (II) et un cocatalyseur consistant en au moins un acide de Lewis.

12. Procédé selon la revendication 11, **caractérisé en ce que** les composés nitriles à insaturation éthylénique sont choisis parmi les nitriles aliphatiques à insaturation éthylénique comprenant les pentènenitriles linéaires comme le pentène-3-nitrile, le pentène-4-nitrile et leurs mélanges.

13. Procédé selon l'une des revendications 11 ou 12, **caractérisé en ce que** l'acide de Lewis mis en oeuvre comme cocatalyseur est choisi parmi les composés des éléments des groupes Ib, IIb, IIIa, IIIb, IVa, IVb, Va, Vb, Vlb, Vllb et VIII de la Classification périodique des éléments.

14. Procédé selon l'une des revendications 11 à 13, **caractérisé en ce que** l'acide de Lewis est choisi parmi le chlorure de zinc, le bromure de zinc, l'iodure de zinc, le chlorure de manganèse, le bromure de manganèse, le chlorure de cadmium, le bromure de cadmium, le chlorure stanneux, le bromure stanneux, le sulfate stanneux, le tartrate stanneux, le trifluorométhylsulfonate d'indium, les chlorures ou bromures des éléments des terres rares comme le lanthane, le cérium, le praséodyme, le néodyme, le samarium, l'europium, le gadolinium, le terbium, le dysprosium, l'hafnium, l'erbium, le thallium, l'ytterbium et le lutétium, le chlorure de cobalt, le chlorure ferreux, le chlorure d'yttrium et leurs mélanges, les composés organométalliques.

15. Procédé selon l'une des revendications 1 à 14, **caractérisé en ce que** l'on réalise en absence de cyanure d'hydrogène l'isomérisation en pentènenitriles, du méthyl-2-butène-3-nitrile présent dans le mélange réactionnel provenant de l'hydrocyanation du butadiène, en opérant en présence d'un catalyseur comportant au moins un composé de formule (II) et au moins un composé d'un métal de transition.

## Patentansprüche

1. Verfahren zur Hydrocyanierung einer Kohlenwasserstoffverbindung mit mindestens einer ethylenischen Ungesättigtheit durch Umsetzung mit Cyanwasserstoff in flüssigem Medium in Gegenwart eines Katalysators, der ein unter den Übergangsmetallen ausgewähltes Metallelement und einen Organophosphorliganden umfasst, **dadurch gekennzeichnet, dass** der Organophosphorligand mindestens eine Verbindung umfasst, die der nachstehenden allgemeinen Formel (II) entspricht: 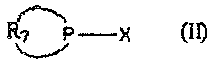 worin:
R₇ für einen zweiwertigen Rest der folgenden allgemeinen Formel (III): 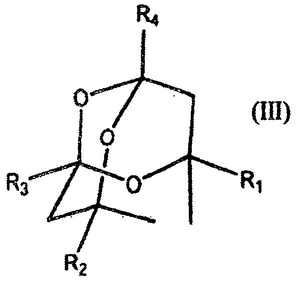 oder einen zweiwertigen Rest der folgenden allgemeinen Formel (IV): 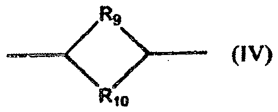 steht, worin R₉ und R₁₀ gleich oder verschieden sind und für einen linearen oder verzweigten aliphatischen zweiwertigen Rest mit 1 bis 6 Kohlenstoffatomen stehen,
R₁, R₂, R₃ und R₄ gleich oder verschieden sind und für ein Wasserstoffatom, einen linearen oder verzweigten Alkylrest mit 1 bis 12 Kohlenstoffatomen, der Heteroatome enthalten kann, einen Rest, der einen gegebenenfalls substituierten aromatischen oder cycloaliphatischen Rest umfasst, der Heteroatome umfassen kann, einen Carbonyl-, Alkoxycarbonyl- oder Alkoxyrest, ein Halogenatom, eine Nitrilgruppe oder eine Halogenalkylgruppe mit 1 bis 12 Kohlenstoffatomen stehen,
X für ein Halogenatom aus der Gruppe umfassend Fluor und Brom steht.

2. Verfahren nach Anspruch 1, **dadurch gekennzeichnet, dass** R₁, R₂, R₃ und R₄ gleich oder verschieden sind und für ein Wasserstoffatom oder einen linearen oder verzweigten Alkylrest mit 1 bis 12 Kohlenstoffatomen, der Heteroatome enthalten kann, stehen.

3. Verfahren nach einem der vorhergehenden Ansprüche, **dadurch gekennzeichnet, dass** die Verbindung der allgemeinen Formel (II) einer der folgenden Formeln entspricht:

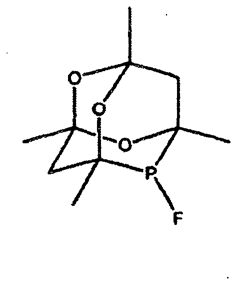

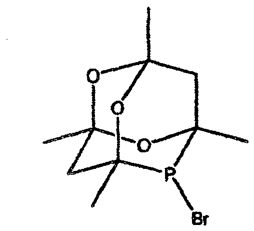

4. Verfahren nach einem der Ansprüche 1 oder 2, **dadurch gekennzeichnet, dass** die Verbindung der allgemeinen Formel (II) einer der folgenden Formeln entspricht:

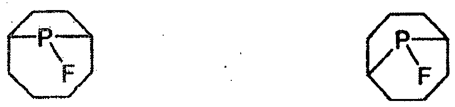

5. Verfahren nach einem der vorhergehenden Ansprüche, **dadurch gekennzeichnet, dass** man das Metallelement aus der Gruppe bestehend aus Nickel, Cobalt, Eisen, Ruthenium, Rhodium, Palladium, Osmium, Iridium, Platin, Kupfer, Silber, Gold, Zink, Cadmium und Quecksilber auswählt.

6. Verfahren nach einem der vorhergehenden Ansprüche, **dadurch gekennzeichnet, dass** die Zusammensetzung des Katalysatorsystems der allgemeinen Formel (V) entspricht:
M[L_{f}]ₜ (V)
worin:
M für ein Übergangsmetall steht,
L_{f} für den Organophosphorliganden bzw. die Organophosphorliganden steht, wovon mindestens einer einer Verbindung der Formel (II) entspricht, t für eine Zahl zwischen 1 und 10 (einschließlich der Grenzwerte) steht.

7. Verfahren nach Anspruch 6, **dadurch gekennzeichnet, dass** L_{f} für eine Mischung von Organophosphor-liganden, umfassend mindestens einen Liganden, der einer Verbindung der Formel (II) entspricht, und mindestens einen einzähnigen Organophosphitliganden, steht.

8. Verfahren nach Anspruch 7, **dadurch gekennzeichnet, dass** man den einzähnigen Organophosphitliganden aus der Gruppe umfassend Tritolylphosphit und Triphenylphosphit auswählt.

9. Verfahren nach einem der vorhergehenden Ansprüche, **dadurch gekennzeichnet, dass** man die organischen Verbindungen mit mindestens einer ethylenischen Doppelbindung aus Diolefinen wie Butadien, Isopren, 1,5-Hexadien, 1,5-Cyclooctadien, ethylenisch ungesättigten aliphatischen Nitrilen, insbesondere linearen Pentennitrilen wie 3-Pentennitril und 4-Pentennitril, Monoolefinen wie Styrol, Methylstyrol, Vinylnaphthalin, Cyclohexen, Methylcyclohexen und Mischungen mehrerer dieser Verbindungen auswählt.

10. Verfahren nach einem der vorhergehenden Ansprüche, **dadurch gekennzeichnet, dass** man die verwendete Menge der Nickelverbindung oder eines anderen Übergangsmetalls so wählt, dass pro Mol zu hydrocyanierende organische Verbindung zwischen 10⁻⁴ und 1 mol verwendetes Nickel oder anderes Übergangsmetall vorliegen, und die verwendete Menge der Organophosphorverbindungen so wählt, dass die Zahl der Mole dieser Verbindungen, bezogen auf 1 mol Übergangsmetall, 0,5 bis 100 beträgt.

11. Verfahren nach einem der vorhergehenden Ansprüche zur Hydrocyanierung von Nitrilverbindungen mit ethylenischer Ungesättigtheit zu Dinitrilen durch Umsetzung mit Cyanwasserstoff, **dadurch gekennzeichnet, dass** man in Gegenwart eines Katalysatorsystems, das mindestens eine Übergangsmetallverbindung, mindestens eine Verbindung der Formel (II) und einen Cokatalysator, der mindestens aus einer Lewis-Säure besteht, arbeitet.

12. Verfahren nach Anspruch 11, **dadurch gekennzeichnet, dass** man die Nitrilverbindungen mit ethylenischer Ungesättigtheit aus aliphatischen Nitrilen mit ethylenischer Ungesättigtheit, die lineare Pentennitrile wie 3-Pentennitril, 4-Pentennitril und Mischungen davon umfassen, auswählt.

13. Verfahren nach einem der Ansprüche 11 oder 12, **dadurch gekennzeichnet, dass** man die als Cokatalysator verwendete Lewis-Säure aus den Verbindungen der Gruppen Ib, IIb, IIIa, IIIb, IVa, IVb, Va, Vb, VIb, VIIb und VIII des Periodensystems der Elemente auswählt.

14. Verfahren nach einem der Ansprüche 11 bis 13, **dadurch gekennzeichnet, dass** man die Lewis-Säure aus Zinkchlorid, Zinkbromid, Zinkiodid, Manganchlorid, Manganbromid, Cadmiumchlorid, Cadmiumbromid, Zinn(II)-chlorid, Zinn(II)-bromid, Zinn(II)-sulfat, Zinn(II)-tartrat, Indiumtrifluormethylsulfonat, den Chloriden oder Bromiden der Seltenerdmetalle wie Lanthan, Cer, Praseodym, Neodym, Samarium, Europium, Gadolinium, Terbium, Dysprosium, Hafnium, Erbium, Thallium, Ytterbium und Lutetium, Cobaltchlorid, Eisen(II)-chlorid, Yttriumchlorid und Mischungen davon und organometallverbindungen auswählt.

15. Verfahren nach einem der Ansprüche 1 bis 14, **dadurch gekennzeichnet, dass** man das in der Reaktionsmischung vorliegende 2-Methyl-3-buten-nitril, das aus der Hydrocyanierung von Butadien stammt, in Abwesenheit von Cyanwasserstoff und in Gegenwart eines Katalysators, der mindestens eine Verbindung der Formel (II) und mindestens eine Übergangsmetallverbindung enthält, zu Pentennitrilen isomerisiert.

## Claims

1. Process for the hydrocyanation of a hydrocarbon-based compound comprising at least one ethylenic unsaturation, by reaction in a liquid medium with hydrogen cyanide in the presence of a catalyst comprising a metal element chosen from transition metals and an organophosphorus ligand, **characterized in that** the organophosphorus ligand comprises at least one compound corresponding to general formula (II): 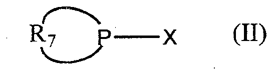 in which:
R₇ represents a divalent radical of general formula (III) below: 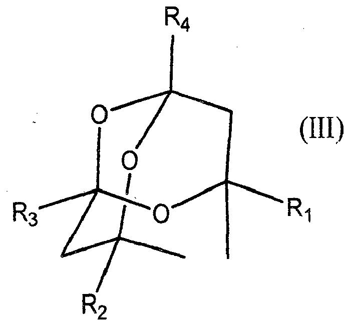 or a divalent radical of general formula (IV) below: 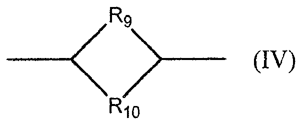 in which R₉ and R₁₀, which may be identical or different, represent a linear or branched, aliphatic divalent radical containing from 1 to 6 carbon atoms, R₁, R₂, R₃ and R₄, which may be identical or different, represent a hydrogen atom, a linear or branched alkyl radical containing from 1 to 12 carbon atoms that may contain heteroatoms, a radical comprising a substituted or unsubstituted aromatic or cycloaliphatic radical which may comprise heteroatoms, a carboxyl, alkoxycarbonyl or alkoxy radical, a halogen atom, a nitrile group or a haloalkyl group containing from 1 to 12 carbon atoms,
X represents a halogen atom selected from the group consisting of fluorine and bromine.

2. Process according to Claim 1, **characterized in that** R₁, R₂, R₃ and R₄, which may be identical or different, represent a hydrogen atom, or a linear or branched alkyl radical containing from 1 to 12 carbon atoms that may contain heteroatoms.

3. Process according to either of the preceding claims, **characterized in that** the compound of general formula (II) corresponds to either of the following formulae:

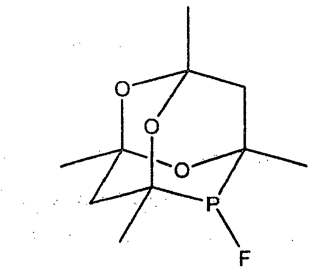

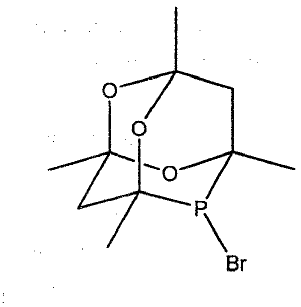

4. Process according to either of Claims 1 and 2, **characterized in that** the compound of general formula (II) corresponds to either of the following formulae:

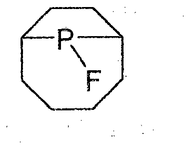

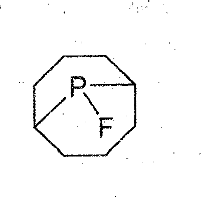

5. Process according to one of the preceding claims, **characterized in that** the metal element is selected from the group consisting of nickel, cobalt, iron, ruthenium, rhodium, palladium, osmium, iridium, platinum, copper, silver, gold, zinc, cadmium and mercury.

6. Process according to one of the preceding claims, **characterized in that** the composition of the catalytic system is expressed by general formula (V):
M [L_{f}]ₜ (V)
in which:
M is a transition metal,
L_{f} represents the organophosphorus ligand(s), at least one of which corresponds to a compound of formula (II),
t represents a number between 1 and 10 (limits included).

7. Process according to Claim 6, **characterized in that** L_{f} represents a mixture of organophosphorus ligands comprising at least one ligand corresponding to a compound of formula (I) or (II) and at least one monodentate organophosphite ligand.

8. Process according to Claim 7, **characterized in that** the monodentate organophosphite ligand is selected from the group consisting of tritolyl phosphite and triphenyl phosphite.

9. Process according to one of the preceding claims, **characterized in that** the organic compounds comprising at least one ethylenic double bond are chosen from diolefins such as butadiene, isoprene, 1,5-hexadiene, 1,5-cyclooctadiene, ethylenically unsaturated aliphatic nitriles, in particular linear pentenenitriles such as 3-pentenenitrile or 4-pentenenitrile, monoolefins such as styrene, methylstyrene, vinylnaphthalene, cyclohexene or methylcyclohexene, and also mixtures of several of these compounds.

10. Process according to one of the preceding claims, **characterized in that** the amount of compound of nickel or of another transition metal used is chosen such that there is, per mole of organic compound to be hydrocyariated, between 10⁻⁴ and 1 mol of nickel or of the other transition metal used, and **in that** the amount of organophosphorus compounds used is chosen such that the number of moles of these compounds with respect to 1 mole of transition metal is from 0.5 to 100.

11. Process according to one of the preceding claims, for the hydrocyanation of ethylenically unsaturated nitrite compounds so as to give dinitriles, by reaction with hydrogen, cyanide, **characterized in that** the reaction is carried out in the presence of a catalytic system comprising at least one compound of a transition metal, at least one compound of formula (II) and a cocatalyst consisting of at least one Lewis acid.

12. Process according to Claim 11, **characterized in that** the ethylenically unsaturated nitrile compounds are chosen from ethylenically unsaturated aliphatic nitriles comprising linear pentenenitriles, such as 3-pentenenitrile, 4-pentenenitrile, and mixtures thereof.

13. Process according to either of Claims 11 and 12, **characterized in that** the Lewis acid used as cocatalyst is chosen from the compounds of the elements from groups Ib, IIb, IIIa, IIIb, IVa, IVb, Va, Vb, VIb, VIIb and VIII of the Periodic Table of Elements.

14. Process according to one of Claims 11 to 13, **characterized in that** the Lewis acid is chosen from zinc chloride, zinc bromide, zinc iodide, manganese chloride, manganese bromide, cadmium chloride, cadmium bromide, stannous chloride, stannous bromide, stannous sulphate, stannous tartrate, indium trifluoromethylsulphonate, the chlorides or bromides of rare-earth elements, such as lanthanum, cerium, praseodymium, neodymium, samarium, europium, gadolinium, terbium, dysprosium, hafnium, erbium, thallium, ytterbium and lutetium, cobalt chloride, ferrous chloride, yttrium chloride and mixtures thereof, and organometallic compounds.

15. Process according to one of Claims 1 to 14, **characterized in that** the isomerization, so as to give pentenenitriles, of the 2-methyl-3-butenenitrile present in the reaction mixture originating from the hydrocyanation of butadiene is carried out in the absence of hydrogen cyanide, the isomerization being carried out in the presence of a catalyst comprising at least one compound of formula (II) and at least one compound of a transition metal.
